# EUROPEAN PATENT SPECIFICATION

(11) **EP 4 373 388 B1**
(45) Date of publication and mention of the grant of the patent: **18.06.2025**
(21) Application number: 22768485.9
(22) Date of filing: 21.07.2022
(51) Int. Cl.: G16H 30/20, G16H 30/40, G16H 50/20, A61B 5/00, G06F 3/01, G16H 40/63, G16H 50/30

(54) **A SYSTEM FOR COMMUNICATION WITH A PATIENT BASED ON THE DETERMINED SEDATION STATE**
SYSTEM ZUR KOMMUNIKATION MIT EINEM PATIENTEN AUF DER BASIS DES BESTIMMTEN SEDIERUNGSZUSTANDS
SYSTÈME DE COMMUNICATION AVEC UN PATIENT FONDÉ SUR L'ÉTAT DE SÉDATION DÉTERMINÉ

(30) Priority: 22.07.2021 IL 28507121; 19.05.2022 IL 29314922
(43) Date of publication of application: 29.05.2024
(73) Proprietor: Eyefree Assisting Communication Ltd., 6618003 Tel Aviv (IL)
(72) Inventor: RETZKIN, Or, 6789037 Tel Aviv (IL); KORNBERG, Itai, 8533800 Lehavim (IL)
(74) Representative: Plasseraud IP
(86) International application number: PCT/IL2022/050789
(87) International publication number: WO 2023/002488

(56) References cited:
- WO-A1-2021/024257
- US-A1- 2007 273 611
- JU XIN-XING ET AL: "A systematic review on voiceless patients' willingness to adopt high-technology augmentative and alternative communication in intensive care units", INTENSIVE AND CRITICAL CARE NURSING, CHURCHILL LIVINGSTONE, EDINBURGH, GB, vol. 63, 7 November 2020 (2020-11-07), XP086526590, ISSN: 0964-3397, [retrieved on 20201107], DOI: 10.1016/J.ICCN.2020.102948

## Description

### TECHNOLOGICAL FIELD

The present disclosure is in the field of automated system for monitoring cognitive state of a patient and communicating with him/her.

### BACKGROUND ART

US 2007/273611 A1 relates generally to apparatus, systems, and methods for monitoring movement of a human eye, e.g., for monitoring fatigue, purposeful communication, and/ or controlling devices based upon movement of an eye, eyelid, and/or other components of the eye or eyes of a person.

Further references considered to be relevant as background to the presently disclosed subject matter are listed below:
- WO 2021/024257;
- WO 2016/142933;
- WO 2019/111257.

Acknowledgement of the above references herein is not to be inferred as meaning that these are in any way relevant to the patentability of the presently disclosed subject matter.

### SUMMARY OF THE INVENTION

The present invention provides a system for interacting with a patient, defined by appended independent claim 1. Favourable embodiments of the system are set out in dependent claims 2-9.

No method is claimed.

### GENERAL DESCRIPTION

A first part of the present disclosure provides a system and a method for interacting with a patient that can be found in a plurality of sedation/cognitive states, ranging from non-responsive states to fully responsive. The terms sedation state and cognitive state are interchangeable, and they both refer to the awareness state of the patient and his/her ability to communicate with the surrounding. The system and the method provide a solution that is adapting the type of communication provided to the patient according to his/her sedation state. This is done to ensure that the communication is effective and there is a reasonable chance that the patient receives and able to process the communication, and in times to respond it. Such adapted communication has much more chance to improve the sedation state of the patient.

Therefore, an aspect of the present disclosure provides a system for interacting with a patient. The system comprising a communication module configured to operate in at least three communication modes that comprises: (i) unidirectional mode, in which the communication module outputs communication to the patient that does not require his/her response. This communication is relevant when the patient is in a sedation state with no capability to respond; (ii) responsive mode, in which the communication module outputs communication to the patient the requires his/her response. This may include questions to or requests from the patient. This communication is relevant when the patient has the capability to respond to communication but lacks the capability to initiate the communication; and (iii) open communication mode, in which the communication module allows the patient to proactively initiate communication with the system. This communication can be in various forms, for example the patient may request from the system to play music, to communicate with clinicians, caregivers, family members, to watch video or the like.

The system further comprising a processing circuitry that comprises an input module configured to receive input data indicative of the patient sedation state. The processing circuitry is configured to (1) determine the sedation state of the patient based on the input data; and (2) triggering the communication module to operate in a selected communication mode in response to the determined sedation state and output a selected communication scheme based thereon.

The following are various embodiments of the system. It is appreciated that, unless specifically stated otherwise, certain features of the presently disclosed subject matter, which are, for clarity, described in the context of separate embodiments, may also be provided in any combination in a single embodiment.

In some embodiments of the system, the communication module is configured to perform and/or allow the patient (i) audible communication, namely output a communication protocol to the patient audibly and also being capable to receive audio commands, (ii) video-based communication, namely to output a video to the patient on a screen, (iii) eye-based communication, namely a camera that records the eyes of the patient and the system classify the eye movements of the patient to distinguished gesture that allows communication with the system, (iv) touch-screen based communication, namely the patient can communicate through a touch screen, (v) tactile communication, (vi) EEG-based communication, namely communication that is based on detection of EEG signals of the patient, e.g. signals received by an EEG unit coupled to the head of the patient, (vii) EOG-based communication, namely communication that is based on detection of EOG signals of the patient, (viii) automatic lip-reading communication, i.e. a camera that records images of the lips of the patient and the processing circuitry is configured to determine from these lips images the words pronounced by the patient and record it as communication of the patient, (ix) head gestures-based communication, i.e. a camera that records images of the head of the patient and the processing circuitry is configured to determine from these head images head gestures that are classified into specific communication performed by the patient, (x) or any combination thereof. These communications are allowed as part of the outputted communication to the patient in response to the determined sedation state.

In some embodiments of the system, the determination of the sedation state of the patient based on the input data comprises classifying the sedation state of the patient into at least three sedation levels, each level triggers one or more level-specific communication modes.

In some embodiments of the system, said classifying comprises scoring the sedation state of the patient, the score defines the sedation level. There are at least three ranges of scores, each range defines a different level. It is to be noted that two level can have overlapping scores.

In some embodiments of the system, the scoring is a Richmond Agitation-Sedation Scale (RASS) score. For example, score of -2 or less and +3 and above triggers the unidirectional mode, a score between and including -1 to +2 triggers the responsive mode and a score between and including 0 to +1, which overlaps with the second range score, triggers an open communication mode. The open communication mode can be operated alone or in combination with the responsive mode in a certain communication protocol profile.

In some embodiments of the system, the processing circuitry is configured to apply at least one or any combination of the following:
- trigger the unidirectional mode upon determining a non-responsive sedation state of the patient;
- trigger the responsive mode and/or the unidirectional mode upon determining a low-level responsive sedation state of the patient;
- trigger the open communication mode and/or the responsive mode and/or the unidirectional mode upon determining a high-level responsive sedation state of the patient.

In some embodiments of the system, the selection of the communication mode is intended to improve the sedation state of the patient.

In some embodiments of the system, the sedation state of the patient is indicative of or the actual delirium state of the patient.

In some embodiments of the system, said input data comprises recorded communication of the patient with the communication module.

In some embodiments of the system, said input data comprises eye image data indicative of recorded images of an eye of the patient.

In some embodiments, the system comprising a camera unit configured for recording images of an eye of the patient and generating eye image data based thereon, wherein said input data comprises said image data.

In some embodiments of the system, said input data comprises EEG data indicative of recorded EEG signals of the patient.

In some embodiments, the system comprising an EEG unit configured for recording EEG signals of the patient and generate EEG data based thereon, said input data comprises said EEG data.

Yet another aspect of the present disclosure provides a method for interacting with a patient. The method comprising
input data indicative of the patient sedation state;
determining based on the input data the sedation state of the patient; and
in response to the determined sedation state of the patient, outputting a selected communication in a communication mode selected from at least one of three communication modes that comprises: unidirectional mode, responsive mode, and open communication mode. These three modes are defined above with respect to the system aspect and these definitions also apply here.

In some embodiments of the method, said selected communication is any one of audible communication, video-based communication, eye-based communication, touchscreen-based communication, tactile communication, EEG-based communication, EOG-based communication, automatic lip-reading communication, head gestures-based communication, or any combination thereof.

In some embodiments of the method, the determination of the sedation state of the patient based on the input data comprises classifying the sedation state of the patient into at least three sedation levels, each level triggers one or more level-specific communication modes.

In some embodiments of the method, said classifying comprises scoring the sedation state of the patient, the score defines the sedation level. There are at least three ranges of scores, each range defines a different level. It is to be noted that two level can have overlapping scores.

In some embodiments of the method, the scoring is a Richmond Agitation-Sedation Scale (RASS) score. For example, score of -2 or less and +3 and above triggers the unidirectional mode, a score between and including -1 to +2 triggers the responsive mode and a score between and including 0 to +1, which overlaps with the second range score, triggers an open communication mode. The open communication mode can be operated alone or in combination with the responsive mode in a certain communication protocol profile.

In some embodiments of the method, said outputting comprising at least one or any combination of the following:
- selecting the unidirectional mode upon determining a non-responsive sedation state of the patient;
- selecting the responsive mode and/or the unidirectional mode upon determining a low-level responsive sedation state of the patient;
- selecting the open communication mode and/or the responsive mode and/or the unidirectional mode upon determining a high-level responsive sedation state of the patient.

In some embodiments of the method, the selection of the communication mode is intended to improve the sedation state of the patient.

In some embodiments of the method, the sedation state of the patient is indicative of or the actual delirium state of the patient.

In some embodiments of the method, said input data comprises recorded communication of the patient with the communication module.

In some embodiments of the method, said input data comprises eye image data indicative of recorded images of an eye of the patient.

In some embodiments of the method, said input data comprises EEG data indicative of recorded EEG signals of the patient.

Another part of the present disclosure provides a system for monitoring the sedation/cognitive state of a patient by continuously monitoring the patient's eye activity and generating eye image date based thereon. The monitor is further configured to provide a selected output to the patient, such as a questionnaire, an audible output and/or a visual output, in order to increase his/her awareness and reduce risks or state of delirium.

Optionally, the system is configured to receive EEG data indicative of recorded EEG signals of the patient that are time-correlated with the recorded eye activity of the patient, and the sedation state of the patient is determined based on either the EEG data, the eye image data or combination thereof. Different sedation states of the patient can be determined by applying different weight factors profiles of the two sets of data.

Upon determination of the sedation state of the patient, the processing circuitry, i.e. the processor/controller of the system, is configured to operate a communication module so as to trigger a selected output of engaging communication to the patient. The output may be interactive, namely one that requires a response from the patient, or passive that only needs to be received by one of the senses of the patient without any required response therefrom. The outputted communication is intended to stimulate the cognitive activity of the patient and thereby improving his/her cognitive state.

Thus, as aspect of the present disclosure provides a system for monitoring sedation level of a patient. The system includes (1) a camera unit configured for recording images of an eye of the patient and generating eye image data based thereon; (2) a communication module operable to output a desired communication protocol; and (3) a processing circuitry. The processing circuitry comprises an input module configured to receive EEG data indicative of EEG signal of the patient and is in data communication with the camera. The processing circuitry is configured to: (i) receive and process said eye image data and EEG data; (ii) determine based on at least one of the eye image data and EEG data the sedation or cognitive state of the user; and (iii) triggering the communication module to output a selected communication protocol in response to the determined sedation state. The communication protocol can be a questionnaire, playing of music, outputting recorded audio of family or friend, etc.

Yet another aspect of the present disclosure provides a system for monitoring sedation level of a patient. The system comprising (1) a camera unit configured for recording images of an eye of the patient and generating eye image data based thereon; (2) a communication module operable to output a desired communication protocol; and (3) a processing circuitry. The processing circuitry is in data communication with the camera and is operable to (i) receive and process said eye image data; (ii) determine based on the eye image data the sedation or cognitive state of the user; and (iii) triggering the communication module to output a selected communication protocol in response to the determined sedation state. The communication protocol can be a questionnaire, playing of music, outputting audio of family or friends, etc.

The following are optional embodiments for any of the above-described aspects. It is appreciated that, unless specifically stated otherwise, certain features of the presently disclosed subject matter, which are, for clarity, described in the context of separate embodiments, may also be provided in any combination in a single embodiment.

In some embodiments, the system further comprising an EEG unit configured for recording EEG signals of the patient and generate said EEG data based thereon.

It is to be noted that any combination of the described embodiments with respect to any aspect of this present disclosure is applicable. In other words, any aspect of the present disclosure can be defined by any combination of the described embodiments.

In some embodiments of the system, the processing circuitry is configured to calculate a sedation score of the patient, such as Richmond Agitation-Sedation Scale (RASS) score, and to classify the score into two or more score ranges, each range triggers a different communication protocol.

In some embodiments of the system, in at least one range of scores, the determination of the sedation state and/or the communication protocol is triggered only based on the EEG data. Thus, in a score that indicates that the patient is sedated and there is no eye activity that can be monitored by the camera unit, the sedation state of the patient is determined only based on the EEG data.

In some embodiments of the system, in at least one range of scores the determination of the sedation state and/or the communication protocol is triggered based on a combination of the eye image data and the EEG data. Namely, in a score that indicates that the patient is alerted at some level and there is eye activity that can be monitored by the camera unit, the determination of the sedation state of the patient is determined based on a certain level of combination of the two data sets. Depending on the recorded activity of the eye and the brain of the patient, the influence of each data for determining the sedation is determined by the processing unit. Typically, when the patient is responsive to some extent and there is eye activity, the eye image data is more significant for the determination of the sedation state.

In some embodiments of the system, the processing circuitry is configured to determine the sedation state of the user by continuously classifying the recorded eye activity of the patient into defined gestures. The temporal profile of the eye gestures defines the sedation state of the patient.

In some embodiments of the system, the processing circuitry is configured for applying different and varying weight factors to the EEG data and the eye image data based on the amount of information or the assumed sedation state of the patient. Namely, during a certain time period, the processing circuitry is configured to apply weight factors on the data sets obtained by the EEG sensor, or the image sensor based on the most recent determined sedation state and/or based on the amount of varying information received by the EEG sensor or image sensor in the certain time period.

In some embodiments of the system, the processing circuitry is configured to apply weight factors on the EEG data and the eye image data based on the recent determined sedation state and update the weight factors when a change of the sedation state is identified. Therefore, when the patient is in a sedation state in which the eye activity is negligible, the weight factors on the EEG data are much more significant and in a sedation state in which there is a significant eye activity by the user, the weight factor of the eye image data increases significantly.

In some embodiments of the system, the processing circuitry is configured to apply a temporal analysis on the eye image data and the EEG data to determine a correlation between eye movements, brain activity and sedation level. This can be performed by applying a machine learning algorithm and training the system by inputting the sedation score level at different scenarios of eye movements and brain activity.

In some embodiments of the system, the processing circuitry is configured to analyze selected time-windows of the eye image data and/or the EEG data following an output of communication protocol to identify patient's response to said communication protocol and to determine an updated sedation state of the patient based on said response.

In some embodiments of the system, the processing circuitry is further configured to transmit a signal carrying sedation state data indicative of the sedation state of the patient to a remote unit. This can be performed through a transmitting unit of the processing circuitry.

In some embodiments of the system, the processing circuitry is further configured to identify specific eye gestures in said eye image data. The eye gestures either trigger a communication protocol or affecting a selected communication protocol. Namely, the identified eye gesture can be used for execution commands in the system and/or to be used to analyze the sedation state of the patient to adapt the relevant communication protocol.

Throughout the specification, an eye gesture should be interpreted as an identified gesture of the eye out of many possible eye gestures. For example, an eye gesture can be a movement of the iris to a certain direction (up, down, right, or left), blinking, steady gaze direction, round movement of the iris, sequence of specific eye gestures, etc.

In some embodiments of the system, the processing circuitry is configured to classify the eye gestures into responses of the patient to a questionnaire. For example, the questionnaire can be Confusion Assessment Method for the Intensive Care Unit (CAM-ICU that is outputted to the patient audibly and the patient respond to each question in the questionnaire with a specific eye gesture that indicates a specific response of the patient to the question.

In some embodiments of the system, the processing circuitry is configured to classify the eye gestures as commands for playing audible output. The audible output is selected from certain music and voice recording of relatives, such as greetings of friends and family.

In some embodiments of the system, the processing circuitry is configured to analyze said eye image and EEG data and identify signatures, namely certain temporal pattern of eye activity, brain activity or a combination thereof, which are indicative of clinical state of the patient.

In some embodiments of the system, the processing circuitry is configured to correlate temporal profiles of said eye image data and/or EEG data with predetermined temporal profiles corresponding to a plurality of signatures, which are indicative of a plurality of clinical states, and to identify a correlation that satisfies a certain condition, such as best match or a certain threshold of matching. The predetermined temporal profiles are stored in a predetermined database and the processing circuitry is in data communication with said predetermined database. In some embodiments, the system further includes said predetermined database.

In some embodiments of the system, the processing circuitry is configured to update the database or to store in a memory thereof so as to generate a personalized signature of the patient upon identifying it. The identification of the personalized signature may be from a clinical indication that is inputted to the system or by manual indication inputted by a user that identifies a certain temporal pattern with the clinical state of the patient.

In some embodiments of the system, the clinical condition is selected from at least one of: pain, thirst, hunger, delirium.

Yet another aspect of the present disclosure provides a method for monitoring sedation state of a patient. The method comprising:
receiving and processing (i) eye image data indicative of recorded images of an eye of the patient and (ii) EEG data indicative of EEG signal of the patient;
determining based on at least one of the eye image data and EEG data the sedation state of the user; and
outputting a selected communication protocol in response to the determined sedation state.

In some embodiments, the method further comprising calculating a sedation score of the patient and classifying the score into two or more score ranges, each range triggers a different output of communication protocol.

In some embodiment of the method, in at least one range of scores the determination of the sedation state and/or the communication protocol is triggered only based on the EEG data.

In some embodiments of the method, in at least one range of scores the determination of the sedation state and/or the communication protocol is triggered based on a combination of the eye image data and the EEG data.

In some embodiments, the method comprising determining the sedation state of the user by continuously classifying the recorded eye activity of the patient into defined gestures. The temporal profile of the eye gestures defines the sedation state of the patient.

In some embodiments, the method further comprising applying temporal analysis on the eye image data and the EEG data and determining a correlation between eye movements, brain activity and sedation state.

In some embodiments, the method comprising applying different and varying weight factors to the EEG data and the eye image data based on the amount of information or the assumed sedation state of the patient. Namely, during a certain time period, the method comprises applying weight factors on the data sets obtained by the EEG sensor, or the image sensor based on the most recent determined sedation state and/or based on the amount of varying information received by the EEG sensor or image sensor in the certain time period.

In some embodiments, the method comprising applying weight factors on the EEG data and the eye image data based on the recent determined sedation state and update the weight factors when a change of the sedation state is identified. Therefore, when the patient is in a sedation state in which the eye activity is negligible, the weight factors on the EEG data are much more significant and in a sedation state in which there is a significant eye activity by the user, the weight factor of the eye image data increases significantly.

In some embodiments, the method comprising analyzing selected time-windows of the eye image data and/or the EEG data following an output of communication protocol to identify patient's response to said communication protocol and to determine an updated sedation state of the patient based on said response.

In some embodiments, the method comprising transmitting a signal carrying sedation state data indicative of the sedation state of the patient to a remote unit.

In some embodiments, the method further comprising identifying eye gestures in said eye image data and either triggering a communication protocol or affecting the selected communication protocol based on said eye gestures. Namely, the identified eye gesture can be used for execution commands in the system and/or to be used to analyze the sedation state of the patient to adapt the relevant communication protocol.

In some embodiments of the method, said eye gestures are used for patient's response to a questionnaire.

In some embodiments of the method, said eye gestures are used for playing audible output, said audible output is selected from certain music and voice recording of relatives.

In some embodiments, the method comprising analyzing said eye image and EEG data and identify signatures indicative of clinical state of the patient.

In some embodiments, the method comprising correlating temporal profiles of said eye image data and/or EEG data with predetermined temporal profiles corresponding to a plurality of signatures that are stored in a database and identifying a correlation that satisfies a certain condition.

In some embodiments of the method, the clinical condition is selected from at least one of: pain, thirst, hunger, delirium.

In some embodiments, the method comprising generating a personalized signature of the patient and updating said database or storing in a memory said personalized signature.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:
**Figs. 1A-1D** are block diagrams of different non-limiting embodiments of the system according to an aspect of the present disclosure.
**Figs. 2A-2B** are block diagrams of different non-limiting embodiments of the system according to another aspect of the present disclosure.

### DETAILED DESCRIPTION

The following figures are provided to exemplify embodiments of the present disclosure.

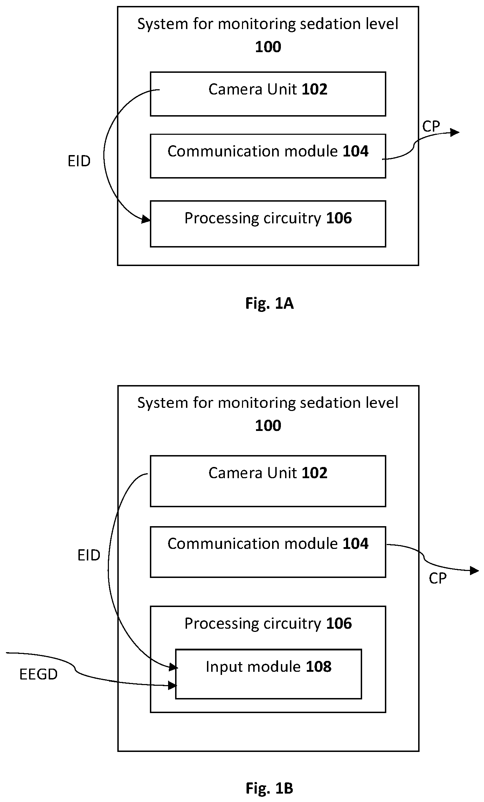

Reference is being made to **Figs. 1A-1D****,** which are non-limiting example of different embodiments of the system for monitoring the sedation level of a patient and engaging the patient with a selected engagement protocol based on the monitored sedation level. It is to be noted that the term "sedation level" is interchangeable with the term "cognitive level", "sedation state", or "cognitive state" and they all refer to a certain sedation/cognitive scale, such as, but not limited to, Richmond Agitation-Sedation Scale (RASS) score. The RASS score indicates what is the patient condition on the scale between "unarousable", in which the patient does not respond to voice or physical stimulation and "combative", in which the patient is fully aware and is overtly combative or violent.

**Fig. 1A** exemplifies a system **100** that includes a camera unit **102** that is configured to continuously image and record the eye of the patient and generate eye image data **EID** indicative of the recorded eye images of the patient. By monitoring the activity of the eye of the patient, the sedation state of the patient can be deduced. This is performed by continuously classifying the recorded eye activity of the patient into defined gestures. The temporal profile of the eye gestures defines the sedation state of the patient.

The system further includes a communication module **104** that is configured to output a selected communication protocol **CP** to the patient in response to the determined sedation state of the patient by the monitoring system **100.** The communication module **104** comprises a plurality of predetermined communication protocols **CPs** and a specific protocol is selected in response to the determined sedation state based on best match criteria. Namely, for each specific sedation state, there is a specific communication protocol. The communication protocol can be tailor-made to the patient, namely that a personalized content is outputted in the communication protocol **CP** to the patient. The communication protocol can include various types of communications, some of them are interactive communications, namely communications that require the patient response, and some of the communication protocols **CPs** are constituted by mere output of the communication module that does not require the patient's response.

A processing circuitry **106** is configured to receive the eye image data **EID** from the camera unit **102** and process it to determine the temporal profile of the eye gestures made by the patient. Based on identification of signatures in the temporal profile of the eye gestures, the sedation level of the patient is determined. Once the sedation level is determined, the processing circuitry is configured to operate the communication module **104** to output a selected communication protocol **CP** to the patient, based on the determined sedation level of the patient. When the communication protocol **CP** is outputted, the camera unit **102** proceeds to record the activity of the eye of the patient and generate eye image data. This new eye image data **EID** is processed by the processing circuitry **106** to determine, by analyzing the temporal profile of the eye gestures made by the patient in a time-window following the output of the communication protocol **CP,** an updated sedation state of the patient and to identify whether the communication protocol **CP** is affecting the sedation state of the patient. By analyzing the response of the patient to communication protocols over time, the processing circuitry can learn how to better match the best communication protocol to the patient to achieve the best progress in the sedation scale of the patient.

**Fig. 1B** is another example of the system, differs from that of **Fig. 1A** by including an input module **108** in the processing circuitry **106.** The input module **108** is configured to receive the eye image data **EID** that is generated by the camera unit **102** and further receive EEG data **EEGD** indicative of EEG signals of the patient that his/her eye activity is being monitored by the system **100.** In this example, the EEG data **EEGD** is generated by an EEG unit that is external to the system **100** and not part of it. The processing circuitry **106** is configured to determine the sedation state of the patient based on the EEG data **EEGD** and the eye image data **EID.** Namely, the processing circuitry is configured to assign different weight factors to each data set (EEG data **EEGD** and the eye image data **EID)** based on the amount of information or the assumed sedation state of the patient. When the patient is at a low sedation score and the eyes of the patient are not responsive, the processing circuitry assigns great weight factors to the EEG data, and when the patient sedations score rises, the weight factors of the eye image data **EID** rise correlatively. Thus, the EEG data **EEGD** is of great importance when the sedation state cannot be determined by analyzing the eye activity of the patient, namely below a certain sedation score. It is to be noted that the processing circuitry is configured to update continuously the weight factors for each patient so as to generate personalized weight factors. In other words, while there are default weight factors at the beginning of the monitoring for each new patient, the processing circuity is configured to update the weight factors to be tailor-made for each patient. The processing circuitry may apply a machine learning algorithm to calculate and update the new weight factors.

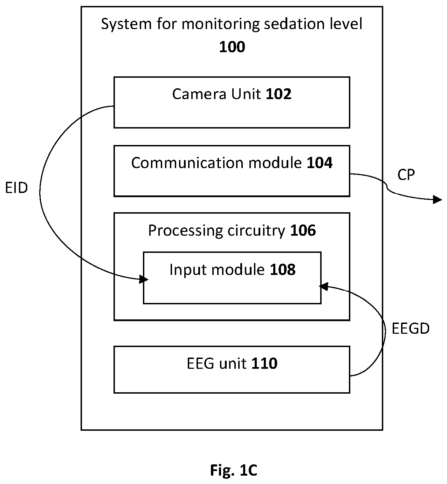

Reference is now being made to **Fig. 1C****,** which is another example of the monitoring system of the present disclosure. This example differs from that of **Fig. 1B** by including the EEG unit **110** that generates the EEG data **EEGD** indicative of EEG signals of the monitored patient. The EEG unit **110** is configured to continuously record the brain waves activity of the patient and generate the EEG data **EEGD** based thereon, which is transmitted to the input module **108** of the processing circuitry **106** to assist in the determination of the sedation level of the patient.

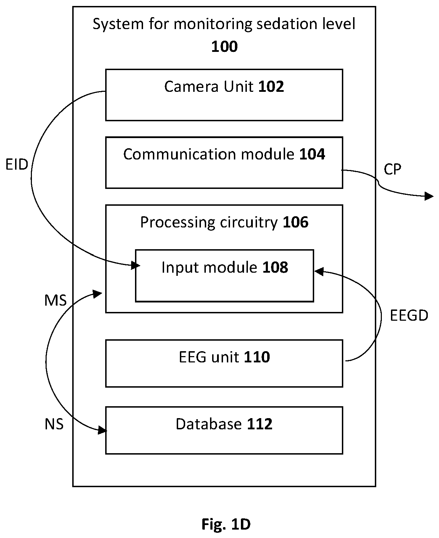

**Fig. 1D** is another example of the monitoring system of the present disclosure. The system of this example differs from that of **Fig. 1C** by including a database **112** that stores a plurality of predetermined signatures of patterns of EEG and eye image data that are each correlated with a specific medical condition of a patient. The predetermined signatures were collected from various patients or were synthetically generated, and each of them is assigned with a medical condition such that the processing circuitry **106** can apply a correlation or matching algorithm on the collected eye image data and EEG data to find a matching signature **MS** in the database and based on that determine the medical condition of the patient. The processing circuitry **106** can record to the database **112** a new signature **NS** that is collected from the monitored patient. It is to be noted that the database **112** may be physically connected to the processing circuitry **106** or can be located on a remote cloud server and communicate with the processing circuitry **106** through standard network protocols.

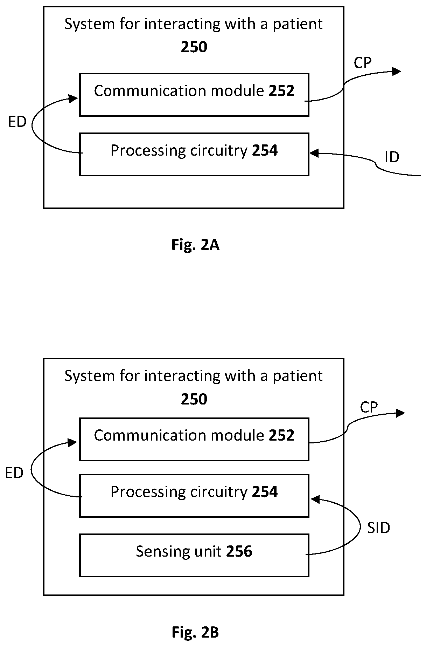

Reference is now being made to **Figs. 2A-2B****,** which are block diagrams of different embodiments of non-limiting examples of a system for interacting with a patient according to an aspect of the present disclosure. **Fig. 2A** shows a system **250** that comprises a communication module **252** and a processing circuitry **254.** The communication module is configured to output a selected communication protocol **CP** in one or more types of communication modes, selected from (i) unidirectional mode, which is a communication that is outputted to the patient without expectation for his/her response, (ii) responsive mode, which is a communication that is outputted to the patient and requires his/her response so the communication module is designed to receive the communication response from the patient (e.g. through a touchscreen, a reading of eyes movement, processing of audible communication, etc.), and open communication mode, which is a communication that is initiated by the patient and being recorded by the communication module and can trigger operations, such as playing music, calling for a caregiver, communicating with a family member, etc.

The processing circuitry **252** is configured to receive input data **ID** being indicative of the sedation state of the patient and processing it to determine the sedation state of the subject. Based on said determination, the processing circuitry transmits execution data **ED** to the communication module **252** to trigger a selected communication protocol **CP** in the selected communication type. In response to receiving the execution data **ED,** the communication protocol outputs the required communication protocol **CP** to the patient.

**Fig. 2B** differs from **Fig. 2A** by showing that the system further comprising a sensing unit **256** that records sensed data of the patient (that can be a record of eye movements, EEG signals, EOG signals, a record of head gestures, microphone, etc.) and generates sensed input data **SID** indicative of the sedation state of the patient and transmits it to the processing circuitry **254** to process the data and determine the sedation state of the patient.

## Claims

1. A system (250) for interacting with a patient, comprising:
a communication module configured to operate in at least three communication modes that comprises: (1) unidirectional mode, in which the communication module outputs communication to the patient that does not require his/her response, (2) responsive mode, in which the communication module outputs communication to the patient that requires his/her response, the responsive mode comprises questions to or requests from the patient, and (3) open communication mode, in which the communication module allows the patient to proactively initiate communication with the system;
a processing circuitry that comprises an input module configured to receive input data indicative of the patient sedation state, the processing circuitry is configured to
i. determine the sedation state of the patient based on the input data;
ii. triggering the communication module to operate in a selected communication mode in response to the determined sedation state;
wherein the determination of the sedation state of the patient based on the input data comprises classifying the sedation state of the patient into one of at least three sedation levels, each level triggers a level-specific communication mode;
wherein said classifying comprises scoring the sedation state of the patient, the score defines the sedation level, wherein three ranges of scores define the sedation levels, each range defines a different level;
wherein the processing circuitry is configured to:
- trigger the unidirectional mode upon determining a non-responsive sedation state of the patient;
- trigger the responsive mode upon determining a low-level responsive sedation state of the patient;
- trigger the open communication mode upon determining a high-level responsive sedation state of the patient.

2. The system of claim 1, wherein the communication module is configured to perform and/or allow the patient audible communication, video-based communication, eye-based communication, touchscreen-based communication, tactile communication, EEG-based communication, EOG-based communication, automatic lip-reading communication, head gestures-based communication, or any combination thereof.

3. The system of claim 1, wherein the scoring is a Richmond Agitation-Sedation Scale (RASS) score.

4. The system of any one of claims 1-3, wherein the sedation state of the patient is the delirium state of the patient.

5. The system of any one of claims 1-4, wherein said input data comprises recorded communication of the patient with the communication module.

6. The system of any one of claims 1-5, wherein said input data comprises eye image data indicative of recorded images of an eye of the patient.

7. The system of any one of claims 1-6, comprising a camera unit configured for recording images of an eye of the patient and generating eye image data based thereon, wherein said input data comprises said image data.

8. The system of any one of claims 1-7, wherein said input data comprises EEG data indicative of recorded EEG signals of the patient.

9. The system of any one of claims 1-8, comprising an EEG unit configured for recording EEG signals of the patient and generate EEG data based thereon, said input data comprises said EEG data.

## Patentansprüche

1. System (250) zum Interagieren mit einem Patienten, umfassend:
ein Kommunikationsmodul, welches dazu eingerichtet ist, in wenigstens drei Kommunikationsmodi zu arbeiten, welche umfassen: (1) unidirektionaler Modus, in welchem das Kommunikationsmodul eine Kommunikation an den Patienten ausgibt, welche nicht seine/ihre Antwort benötigt, (2) ein Reaktionsmodus, in welchem das Kommunikationsmodul eine Kommunikation an den Patienten ausgibt, welche seine/ihre Antwort benötigt, wobei der Reaktionsmodus Fragen an oder Anfragen von dem Patienten umfasst, und (3) ein offener Kommunikationsmodus, in welchem das Kommunikationsmodul dem Patienten erlaubt, proaktiv eine Kommunikation mit dem System zu initiieren;
eine Verarbeitungsschaltung, welche ein Eingabemodul umfasst, welches dazu eingerichtet ist, Eingabedaten zu empfangen, welche für den Patientensedierungszustand bezeichnend sind, wobei die Verarbeitungsschaltung eingerichtet ist, zum
i. Bestimmen des Sedierungszustands des Patienten auf Grundlage der Eingabedaten;
ii. Auslösen des Kommunikationsmoduls, in einem ausgewählten Kommunikationsmodus als Antwort auf den bestimmten Sedierungszustand zu arbeiten;
wobei die Bestimmung des Sedierungszustands des Patienten auf Grundlage der Eingabedaten ein Klassifizieren des Sedierungszustands des Patienten in eine von wenigstens drei Sedierungsstufen umfasst, wobei jede Stufe einen stufenspezifischen Kommunikationsmodus auslöst;
wobei das Klassifizieren ein Bewerten des Sedierungszustands des Patienten umfasst, wobei die Bewertung die Sedierungsstufe definiert, wobei drei Bereiche von Bewertungen die Sedierungsstufen definieren, wobei jeder Bereich eine unterschiedliche Stufe definiert;
wobei die Verarbeitungsschaltung eingerichtet ist, zum:
- Auslösen des unidirektionalen Modus, auf ein Bestimmen eines nichtreagierenden Sedierungszustands des Patienten hin;
- Auslösen des reagierenden Modus, auf ein Bestimmen eines niedrigstufigen reagierenden Sedierungszustands des Patienten hin;
- Auslösen des offenen Kommunikationsmodus, auf ein Bestimmen eines hochstufigen reagierenden Sedierungszustands des Patienten hin.

2. System nach Anspruch 1, wobei das Kommunikationsmodul dazu eingerichtet ist, eine hörbare Kommunikation, eine videobasierte Kommunikation, eine augenbasierte Kommunikation, eine berührungsbildschirmbasierte Kommunikation, eine tastbare Kommunikation, eine EEG-basierte Kommunikation, eine EOG-basierte Kommunikation, eine automatische Lippenlesekommunikation, eine kopfgestenbasierte Kommunikation oder jede Kombination daraus durchzuführen und/oder dem Patienten zu erlauben.

3. System nach Anspruch 1, wobei das Bewerten eine Richmond-Agitation-Sedation-Scale (RASS) Bewertung ist.

4. System nach einem der Ansprüche 1 bis 3, wobei der Sedierungszustand des Patienten ein Deliriumzustand des Patienten ist.

5. System nach einem der Ansprüche 1 bis 4, wobei die Eingabedaten eine aufgenommene Kommunikation des Patienten mit dem Kommunikationsmodul umfassen.

6. System nach einem der Ansprüche 1 bis 5, wobei die Eingabedaten Augenbilddaten umfassen, welche für aufgenommene Bilder eines Auges des Patienten bezeichnend sind.

7. System nach einem der Ansprüche 1 bis 6, umfassend eine Kameraeinheit, welche dazu eingerichtet ist, Bilder eines Auges des Patienten aufzunehmen und Augenbilddaten auf Grundlage davon zu erzeugen, wobei die Eingabedaten die Bilddaten umfassen.

8. System nach einem der Ansprüche 1 bis 7, wobei die Eingabedaten EEG-Daten umfassen, welche für aufgenommene EEG-Signale des Patienten bezeichnend sind.

9. System nach einem der Ansprüche 1 bis 8, umfassend eine EEG-Einheit, welche dazu eingerichtet ist, EEG-Signale des Patienten aufzunehmen und EEG-Daten auf Grundlage davon zu erzeugen, wobei die Eingabedaten die EEG-Daten umfassen.

## Revendications

1. Système (250) d'interaction avec un patient, comprenant :
un module de communication configuré pour fonctionner dans au moins trois modes de communication qui comprend : (1) un mode unidirectionnel, dans lequel le module de communication émet une communication adressée au patient qui ne nécessite pas sa réponse, (2) un mode réactif, dans lequel le module de communication émet une communication adressée au patient qui nécessite sa réponse, le mode réactif comprend des questions adressées au ou des demandes du patient et (3) un mode de communication ouvert, dans lequel le module de communication permet au patient d'initier de manière proactive une communication avec le système ;
un ensemble de circuits de traitement qui comprend un module d'entrée configuré pour recevoir des données d'entrée indiquant l'état de sédation du patient, l'ensemble de circuits de traitement est configuré pour
i. déterminer l'état de sédation du patient sur la base des données d'entrée ;
ii. déclencher le module de communication pour qu'il fonctionne dans un mode de communication sélectionné en réponse à l'état de sédation déterminé ;
dans lequel la détermination de l'état de sédation du patient sur la base des données d'entrée comprend la classification de l'état de sédation du patient dans l'un d'au moins trois niveaux de sédation, chaque niveau déclenche un mode de communication spécifique au niveau ;
dans lequel ladite classification comprend la notation de l'état de sédation du patient, le score définit le niveau de sédation, dans lequel trois plages de scores définissent les niveaux de sédation, chaque plage définit un niveau différent ;
dans lequel l'ensemble de circuits de traitement est configuré pour :
- déclencher le mode unidirectionnel lorsqu'un état de sédation non réactif du patient est déterminé ;
- déclencher le mode réactif lorsqu'un état de sédation réactif de faible niveau du patient est déterminé ;
- déclencher le mode de communication ouvert lorsqu'un état de sédation réactif de haut niveau du patient est déterminé.

2. Système selon la revendication 1, dans lequel le module de communication est configuré pour réaliser et/ou permettre au patient une communication audible, une communication vidéo, une communication oculaire, une communication par écran tactile, une communication tactile, une communication par EEG, une communication par EOG, une communication par lecture labiale automatique, une communication par hochements de tête, ou toute combinaison de celles-ci.

3. Système selon la revendication 1, dans lequel le score est un score d'échelle de vigilance-agitation de Richmond (RASS).

4. Système selon l'une quelconque des revendications 1 à 3, dans lequel l'état de sédation du patient est l'état de délire du patient.

5. Système selon l'une quelconque des revendications 1 à 4, dans lequel lesdites données d'entrée comprennent une communication enregistrée du patient avec le module de communication.

6. Système selon l'une quelconque des revendications 1 à 5, dans lequel lesdites données d'entrée comprennent des données d'images oculaires indiquant des images enregistrées d'un œil du patient.

7. Système selon l'une quelconque des revendications 1 à 6, comprenant une unité caméra configurée pour enregistrer des images d'un œil du patient et générer des données d'images oculaires sur la base de celles-ci, dans lequel lesdites données d'entrée comprennent lesdites données d'image.

8. Système selon l'une quelconque des revendications 1 à 7, dans lequel lesdites données d'entrée comprennent des données EEG indiquant des signaux EEG enregistrés du patient.

9. Système selon l'une quelconque des revendications 1 à 8, comprenant une unité EEG configurée pour enregistrer des signaux EEG du patient et générer des données EEG sur la base de ceux-ci, lesdites données d'entrée comprennent lesdites données EEG.
